# EUROPÄISCHE PATENTSCHRIFT

(11) **EP 2 403 826 B1**
(45) Veröffentlichungstag und Bekanntmachung des Hinweises auf die Patenterteilung: **24.05.2017**
(21) Anmeldenummer: 10706260.6
(22) Anmeldetag: 04.03.2010
(51) Int. Cl.: C07C 263/10, C07C 265/00, B01F 3/02

(54) **VERFAHREN UND VORRICHTUNG ZUR HERSTELLUNG VON ISOCYANATEN**
METHOD AND DEVICE FOR PRODUCING ISOCYANATES
PROCÉDÉ ET DISPOSITIF DE PRÉPARATION D'ISOCYANATES

(30) Priorität: 06.03.2009 EP 09154490
(43) Veröffentlichungstag der Anmeldung: 11.01.2012
(73) Patentinhaber: BASF SE, 67056 Ludwigshafen am Rhein (DE)
(72) Erfinder: KNOESCHE, Carsten, 67150 Niederkirchen (DE); MATTKE, Torsten, 67251 Freinsheim (DE)
(86) Internationale Anmeldenummer: PCT/EP2010/052748
(87) Internationale Veröffentlichungsnummer: WO 2010/100221

(56) Entgegenhaltungen:
- EP-A1- 1 935 876
- EP-A2- 1 754 698
- WO-A1-2008/071564

## Beschreibung

Die Erfindung betrifft ein Verfahren zur Herstellung von Isocyanaten durch Umsetzung der korrespondierenden Amine mit Phosgen in der Gasphase, gegebenenfalls in Gegenwart eines Inertmediums, bei dem das Amin in einem Verdampfer verdampft wird, mit dem Phosgen gemischt wird und das Amin und das Phosgen in einem Reaktor zum Isocyanat umgesetzt werden.

Die Herstellung von Isocyanaten durch Phosgenierung der korrespondierenden Amine kann prinzipiell durch eine Flüssigphasen- oder eine Gasphasenphosgenierung erfolgen. Die Gasphasenphosgenierung zeichnet sich dadurch aus, dass eine höhere Selektivität, ein geringerer Hold-up an toxischem Phosgen sowie ein verminderter Energiebedarf erforderlich sind.

Bei der Gasphasenphosgenierung werden ein aminhaltiger Eduktstrom und ein phosgenhaltiger Eduktstrom jeweils in gasförmigem Zustand vermischt. Das Amin und das Phosgen setzen sich unter Freisetzung von Chlorwasserstoff (HCl) zu den entsprechenden Isocyanaten um. Der aminhaltige Eduktstrom liegt im Allgemeinen in flüssiger Phase vor und muss vor der Vermischung mit dem phosgenhaltigen Strom verdampft und gegebenenfalls überhitzt werden.

Entsprechende Verfahren zur Herstellung von Isocyanaten in der Gasphase sind zum Beispiel in EP-A 1 319 655, EP-A 1 555 258, EP-A 1 935 876 oder EP-A 0 289 840 beschrieben.

Um den aminhaltigen Eduktstrom in der Gasphase mit dem phosgenhaltigen Eduktstrom zu vermischen, ist es notwendig, den aminhaltigen Eduktstrom zunächst zu verdampfen. Ein geeigneter Verdampfer ist zum Beispiel in EP-A 1 754 698 offenbart.

Nachteil der bekannten Verfahren zur Gasphasenphosgenierung ist jedoch, dass sich zwischen Verdampfer und Mischorgan Feststoffablagerungen bilden, die eine Reinigung des Systems notwendig machen. Ursache hierfür ist, dass mit verdampftem Amin in Kontakt stehende Oberflächen lokal oder flächig die Taupunkttemperatur des Gasgemischs unterschreiten. Auf diesen Oberflächen scheidet sich Amin ab, das aufgrund langer Verweilzeiten und hoher Temperaturen langsam vercrackt und so Ablagerungen an den Oberflächen bildet. Diese können zu Belägen aufwachsen oder zu Verstopfungen in nachfolgenden Anlagenteilen, beispielsweise einer Mischdüse, durch Mitriss von festen Bestandteilen führen. Daher ist eine regelmäßige Reinigung erforderlich. Hierzu ist es notwendig, die Anlage abzuschalten. Ablagerungen durch auskondensiertes und vercracktes Amin können zum Beispiel an Wandungen des Verdampfers und an nachfolgenden Rohrleitungen und Apparaturen, beispielsweise Tropfenabscheidern oder Wärmeübertragern entstehen.

Ursache für das Unterschreiten der Taupunktsgrenze an den inneren Oberflächen der Aminzuführung sind zum Beispiel Wärmeverluste in den Rohrleitungen und Apparaten oder das Zumischen von kälteren gasförmigen Inertgasströmen. Die Gefahr einer Kondensatbildung ist insbesondere in der Nähe von Wärmebrücken und in Totzonen der Gasströmung besonders groß. Die Totzonen in der Gasströmung können zum Beispiel durch Messstutzen, beispielsweise für Druckmessungen oder Temperaturmessungen, oder durch Stellorgane wie Hähne oder Ventile entstehen. Feststoffablagerungen bilden sich jedoch auch vermehrt an rauen Oberflächen oder an Spalten oder Ritzen.

Aufgabe der Erfindung ist es daher, ein Verfahren zur Herstellung von Isocyanaten durch Umsetzung der korrespondierenden Amine mit Phosgen in der Gasphase bereitzustellen, durch das vermieden wird, dass Verstopfungen in der Aminzufuhr zum Mischorgan auftreten, die eine Reinigung und damit eine Abschaltung der Anlage notwendig machen.

Gelöst wird die Aufgabe durch ein Verfahren zur Herstellung von Isocyanaten durch Umsetzung der korrespondierenden Amine mit Phosgen in der Gasphase, gegebenenfalls in Gegenwart eines Inertmediums, bei dem das Amin in einem Verdampfer zu einem Amin enthaltenden Gasstrom verdampft wird, das Phosgen in den Amin enthaltenden Gasstrom gemischt wird und das Amin und das Phosgen in einem Reaktor zum Isocyanat umgesetzt werden, bei dem die Temperatur von mit dem gasförmigen Amin in Kontakt stehenden Oberflächen oberhalb der Taupunktsgrenze des Amin enthaltenden Gasstroms gehalten wird.

Mit dem gasförmigen Amin in Kontakt stehende Oberflächen sind Oberflächen von Rohrleitungen, die den Verdampfer mit dem Reaktor verbinden, Zufuhrleitungen, Zufuhrdüsen oder Oberflächen des Verdampfers..

Um die Temperatur der mit dem gasförmigen Amin in Kontakt stehenden Oberflächen oberhalb der Taupunktsgrenze des Amin enthaltenden Gasstroms zu halten, werden Rohrleitungen und Apparaten isoliert, um Wärmeverluste zu minimieren. Zusätzlich ist mindestens eines der folgenden Merkmale erfüllt:
(a) Überhitzen des gasförmigen Aminstroms, um lokale Taupunktsunterschreitungen durch Wärmeverluste auszuschließen,
(b) Beheizen von Rohrleitungen und Apparaten, um Taupunktsunterschreitungen zu verhindern,
(c) Einspeisung eines temperierten Inertgasstroms, wobei die Temperatur des zugeführten Inertgases so eingestellt wird, dass ein Temperaturabstand der Mischtemperatur zur Kondensationsgrenze von mindestens 5 K besteht.

Die Maßnahmen können sowohl einzeln als auch in beliebiger Kombination von zwei oder mehreren umgesetzt werden.

Durch das erfindungsgemäße Verfahren wird das Ausbilden von Ablagerungen an Wandungen der Anlage zur Herstellung von Isocyanaten vermieden oder deutlich reduziert.

Weiterhin können Feststoffablagerungen auch durch konstruktive Maßnahmen verhindert oder vermindert werden.

Als Taupunktsgrenze beziehungsweise Taupunktstemperatur des Amin enthaltenden Gasstroms wird die Temperatur verstanden, bei der bei gegebenem Druck das Amin aus dem Amin enthaltenden Gasstrom auskondensiert.

Eine geeignete Vorrichtung zur Herstellung von Isocyanaten durch Umsetzung der korrespondierenden Amine mit Phosgen in der Gasphase, umfasst einen Verdampfer zur Verdampfung des Amins und einen Reaktor, in dem die Umsetzung erfolgt, sowie Mittel zur Verbindung von Verdampfer und Reaktor. Erfindungsgemäß sind Oberflächen, die mit gasförmigem Amin in Kontakt kommen können, mit einer gegenüber Amin nicht benetzenden Beschichtung versehen, weisen eine gemittelte Rauhtiefe Rz gemäß DIN ISO 4287 von maximal 10 µm auf und/oder die Vorrichtung weist keine Toträume und Wärmebrücken auf. Durch Einsatz einer erfindungsgemäßen Vorrichtung wird die Bildung von Ablagerungen an Anlagenbauteilen, die mit gasförmigem Amin in Kontakt kommen können, beispielsweise Oberflächen des Aminverdampfers, Rohrleitungen, die den Verdampfer mit dem Reaktor verbinden und an Oberflächen des Reaktors, vermieden.

Durch Überhitzen des Aminstroms wird verhindert, dass bei Wärmeverlusten eine Unterschreitung der Taupunktstemperatur erfolgt. Das Überhitzen kann bei entsprechender Auslegung schon im Verdampfer geschehen. Aufgrund der thermischen Zersetzung der Amine bei hohen Temperaturen ist es jedoch bevorzugt, die Überhitzung des Aminstroms möglichst gering zu halten. Durch Isolierung von Rohrleitungen und Apparaten lassen sich die Wärmeverluste minimieren und damit die notwendige Überhitzung klein halten. Als günstig haben sich Temperaturdifferenzen zur Kondensationsgrenze von mindestens 5 K, bevorzugt von mindestens 10 K und besonders bevorzugt von mindestens 20 K erwiesen. Insbesondere das lokale Unterschreiten der Kondensationsgrenze, insbesondere an Wärmebrücken oder in Totzonen der Strömung, wird so vermieden.

Eine weitere sehr wirksame Methode zur Verhinderung von Kondensatanfall an den Oberflächen, die mit gasförmigem Amin in Kontakt stehen, stellt die Begleitbeheizung der Apparate und Rohrleitungen zur Sicherstellung einer Oberflächentemperatur der mit gasförmigem Amin in Kontakt kommenden Flächen oberhalb der Taupunktstemperatur dar. Wärmeverluste können auf diese Weise verhindert oder sogar ausgeglichen werden. Die Begleitbeheizung wird so eingestellt, dass sich eine Oberflächentemperatur von mindestens 5 K, bevorzugt von mindestens 10 K und besonders bevorzugt von mindestens 20 K oberhalb der Kondensationsgrenze einstellt. Die Begleitbeheizung der Oberflächen kann zum Beispiel durch Ausbildung von Wandungen mit einem Doppelmantel oder Aufbringen von Rohrschlangen und Durchströmen mit einem Heizmedium oder durch elektrische Beheizung realisiert werden.

Durch Einspeisen eines temperierten Inertgases in den Aminstrom lässt sich der Partialdruck des Amins senken und damit der Taupunkt des Gasgemisches senken. Als Inertmedien können zum Beispiel Stickstoff, Edelgase wie Helium oder Argon, Aromaten wie Chlorbenzol, o-Dichlorbenzol, Trichlorbenzol, Toluol, Xylol, Chlornaphtalin, Decahydronaphtalin, Kohlenstoffdioxid, Kohlenstoffmonoxid oder Mischungen daraus eingesetzt werden. Bevorzugt werden Stickstoff und/oder Chlorbenzol verwendet. Die Temperatur des zugeführten Inertgases wird so eingestellt, dass ein Temperaturabstand der Mischtemperatur zur Kondensationsgrenze von mindestens 5 K, bevorzugt von mindestens 10 K und besonders bevorzugt von mindestens 20 K besteht.

Durch die Vermeidung des Ausbildens von Ablagerungen durch das erfindungsgemäße Verfahren kann die Standzeit der Anlage zur Herstellung von Isocyanaten gegenüber den aus dem Stand der Technik bekannten Verfahren erhöht werden. Es ist eine weniger häufige Abschaltung zur Reinigung der Anlage notwendig.

Zur Herstellung des Isocyanates werden das Phosgen und das Amin vorzugsweise zunächst einer Mischzone zugeführt, in der die Vermischung von Amin und Phosgen zu einem Reaktionsgemisch erfolgt. Anschließend wird das Reaktionsgemisch dem Reaktor zugeführt, in dem die Umsetzung zum Isocyanat erfolgt. Die Umsetzung von Amin und Phosgen im Reaktor erfolgt dabei in der Gasphase. Hierzu liegt der Druck im Reaktor vorzugsweise im Bereich zwischen 0,3 bis 5 bar absolut, besonders bevorzugt im Bereich von 0,8 bis 3,5 bar absolut. Die Temperatur liegt dabei vorzugsweise im Bereich von 250 bis 550°C, insbesondere im Bereich von 300 bis 500°C.

Um die Reaktion in der Gasphase durchführen zu können, werden das Amin und das Phosgen gasförmig zugegeben. Hierzu weist das Amin vorzugsweise eine Temperatur im Bereich von 200 bis 400°C auf. Der Druck des zugegebenen Amins liegt dabei vorzugsweise im Bereich zwischen 0,05 bis 3 bar absolut. Die Temperatur des zugegebenen Phosgens liegt vorzugsweise im Bereich von 250 bis 500°C. Hierzu wird das Phosgen üblicherweise vor Zugabe auf dem Fachmann bekannte Weise erwärmt.

Zur Erwärmung des Phosgens und des Amins und zur Verdampfung des Amins wird zum Beispiel eine elektrische Beheizung oder eine direkte oder indirekte Aufheizung durch Verbrennung eines Brennstoffs eingesetzt. Als Brennstoffe werden üblicherweise Brenngase, beispielsweise Erdgas, eingesetzt. Durch die Absenkung des Drucks bei der Verdampfung und damit der Siedetemperatur des Amins ist jedoch auch eine Beheizung durch Wasserdampf möglich. In Abhängigkeit von der Siedetemperatur des Amins wird hierbei der Druck des Wasserdampfes ausgewählt. Ein geeigneter Dampfdruck des Wasserdampfes liegt zum Beispiel im Bereich von 40 bis 100 bar. Daraus ergibt sich eine Temperatur des Wasserdampfes im Bereich von 250 bis 311°C.

Im Allgemeinen ist es erforderlich, das Amin mehrstufig auf die Reaktionstemperatur zu erwärmen. Im Allgemeinen wird das Amin hierzu zunächst vorgewärmt, dann verdampft und anschließend überhitzt. Im Allgemeinen erfordert die Verdampfung die längsten Verweilzeiten und führt so zu Zersetzungen des Amins. Um dies zu minimieren, ist eine Verdampfung bei niedrigeren Temperaturen, wie es sich zum Beispiel durch den niedrigeren Druck ergibt, vorteilhaft. Um nach der Verdampfung das verdampfte Amin auf Reaktionstemperatur zu überhitzen, ist im Allgemeinen eine Beheizung mit Wasserdampf nicht ausreichend. Zur Überhitzung wird daher üblicherweise eine elektrische Beheizung oder eine direkte oder indirekte Aufheizung durch Verbrennung eines Brennstoffs eingesetzt.

Aufgrund der hohen Siedetemperaturen des Amins und der daraus resultierenden großen Temperaturdifferenzen zur Umgebung können die Wandungen von Anlagenteilen, beispielsweise von Rohrleitungen, die den Verdampfer mit dem Reaktor verbinden oder von Teilen des Reaktors, beispielsweise Zufuhrleitungen oder Zufuhrdüsen, Temperaturen aufweisen, die unterhalb der Verdampfungstemperatur des Amins liegen. Dies führt dazu, dass an den Wandungen Amin aus dem Gasstrom auskondensieren kann. Auskondensierte Tröpfchen bleiben an der Wandung haften und führen infolge der hohen Temperaturen zu Feststoffbildung und Ablagerungen. Durch solche Ablagerungen werden beispielsweise Rohrquerschnitte verringert und können bei hinreichend kleinen Durchmessern gegebenenfalls vollständig zugesetzt werden. Dies macht eine regelmäßige Reinigung der Anlagenteile notwendig. Durch das erfindungsgemäße Verfahren wird das Entstehen solcher Ablagerungen reduziert oder vermieden, so dass höhere Standzeiten der Anlage erreicht werden können.

Im Unterschied zur Verdampfung des Amins erfolgt die Verdampfung des Phosgens im Allgemeinen bei deutlich niedrigeren Temperaturen. Aus diesem Grund kann zur Verdampfung des Phosgens im Allgemeinen Wasserdampf eingesetzt werden. Jedoch ist auch die notwendige Überhitzung des Phosgens, um dieses auf Reaktionstemperatur zu erwärmen, im Allgemeinen nur durch eine elektrische Beheizung oder eine direkte oder indirekte Aufheizung durch Verbrennung eines Brennstoffes möglich.

Der Reaktor, der zur Phosgenierung des Amins zur Herstellung von Isocyanaten eingesetzt wird, ist dem Fachmann bekannt. Im Allgemeinen werden als Reaktor Rohrreaktoren eingesetzt. Im Reaktor wird das Amin mit dem Phosgen zum korrespondierenden Isocyanat und Chlorwasserstoff umgesetzt. Üblicherweise wird das Phosgen im Überschuss zugegeben, so dass das im Reaktor entstehende Reaktionsgas neben dem gebildeten Isocyanat und dem Chlorwasserstoff auch Phosgen enthält.

Amine, die zur Herstellung von Isocyanaten eingesetzt werden können, sind Monoamine, Diamine, Triamine oder höherwertige Amine. Bevorzugt werden Monoamine oder Diamine eingesetzt. Entsprechend des eingesetzten Amins ergeben sich die korrespondierenden Monoisocyanate, Diisocyanate, Triisocyanate oder höherwertigen Isocyanate. Bevorzugt werden Monoisocyanate oder Diisocyanate mit dem erfindungsgemäßen Verfahren hergestellt.

Diamine und Diisocyanate können aliphatisch, cycloaliphatisch oder aromatisch sein.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

Im Folgenden wird die Bezeichnung (cyclo)aliphatische Isocyanate für cycloaliphatische und/oder aliphatische Isocyanate verwendet.

Beispiele für aromatische Mono- und Diisocyanate sind bevorzugt solche mit 6 bis 20 C-Atomen, beispielsweise Phenylisocyanat, monomeres 2,4'- und/oder 4,4'-Methylendi(phenylisocyanat) (MDI), 2,4- und/oder 2,6-Toluylendiisocyanat (TDI) und 1,5- oder 1,8-Naphthyldiisocyanat (NDI).

Beispiele für (cyclo)aliphatische Diisocyanate sind aliphatische Diisocyanate wie 1,4-Tetramethylendiisocyanat, 1,6-Hexamethylendiisocyanat (1,6-Diisocyanatohexan), 1,8-Octamethylendiisocyanat, 1,10-Decamethylendiisocyanat, 1,12-Dodecamethylendiisocyanat, 1,14-Tetradecamethylendiisocyanat, 1,5-Diisocyanatopentan, Neopentandiisocyanat, Derivate des Lysindiisocyanats, Tetramethylxylylendiisocyanat (TMXDI), Trimethylhexandiisocyanat oder Tetramethylhexandiisocyanat, sowie 3(beziehungsweise 4)-8(beziehungsweise 9)-Bis(isocyanatomethyl)-tricyclo-[5.2.1.0^{2.6}]-decan-Isomerengemische, sowie cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5-trimethyl-5-(isocyanatomethyl)cyclohexan (Isoforondiisocyanat), 1,3- oder 1,4-Bis(isocyanatomethyl)cyclohexan, 2,4- oder 2,6-Diisocyanato-1-methylcyclohexan.

Bevorzugte (cyclo)aliphatische Diisocyanate sind 1,6-Diisocyanatohexan, 1-Isocyanato-3,3,5-trimethyl-5-(isocyanato-methyl)cyclohexan, 4,4'-Di(isocyanatocyclohexyl)methan. Besonders bevorzugt sind 1,6-Diisocyanatohexan, 1-Isocyanato-3,3,5-trimethyl-5-(isocyanatomethyl)cyclohexan und 4,4'-Di(isocyanatocyclohexyl)methan.

Amine, die bei dem erfindungsgemäßen Verfahren zur Reaktion zu den korrespondierenden Isocyanaten eingesetzt werden, sind solche, bei denen das Amin, die korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 Mol-%, besonders bevorzugt zu höchstens 1 Mol-% und ganz besonders bevorzugt zu höchstens 0,5 Mol-% zersetzen. Besonders geeignet sind hier Amine, insbesondere Diamine, auf Basis von (cyclo)aliphatischen Kohlenwasserstoffen mit 2 bis 18 Kohlenstoffatomen. Beispiele hierfür sind 1,6-Diaminohexan, 1,5-Diaminopentan, 1,3-Bis(aminomethyl)cyclohexan, 1-Amino-3,3,5-trimethyl-5-amino-methylcyclohexan (IP-DA) und 4,4-Diaminodicyclohexylmethan. Bevorzugt verwendet wird 1,6-Diaminohexan (H DA).

Ebenfalls können für das erfindungsgemäße Verfahren aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (Mol/Mol)-Gemisch, Diaminobenzol, 2,6-Xylidin, Naphthyldiamin (NDA) und 2,4'- oder 4,4'-Methylen(diphenyldiamin) (MDA) oder Isomerengemische davon. Bevorzugt sind unter diesen die Diamine, besonders bevorzugt sind 2,4- und/oder 2,6-TDA oder 2,4'-und/oder 4,4'-MDA.

Zur Herstellung von Monoisocyanaten können ebenfalls aliphatische, cycloaliphatische oder aromatische Amine, üblicherweise Monoamine, eingesetzt werden. Als aromatisches Monoamin ist insbesondere Anilin bevorzugt.

Bei der Gasphasenphosgenierung ist es anzustreben, dass die im Reaktionsverlauf auftretenden Verbindungen, also Edukte (Amin und Phosgen), Zwischenprodukte (insbesondere die intermediär entstehenden Carbamoyl- und Aminhydrochloride), Endprodukte (Diisocyanat) sowie gegebenenfalls zudosierte inerte Verbindungen, unter den Reaktionsbedingungen in der Gasphase verbleiben. Sollten diese oder andere Komponenten sich aus der Gasphase zum Beispiel an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für Auftreten der Aminhydrochloride, die sich aus freien Aminogruppen und Chlorwasserstoff bilden, da die resultierenden Aminhydrochloride leicht ausfallen und nur schwer wieder verdampfbar sind.

Neben dem Einsatz eines Rohrreaktors ist es auch möglich, im Wesentlichen quaderförmige Reaktionsräume, beispielsweise Plattenreaktoren zu verwenden. Auch jeder beliebige andere Querschnitt des Reaktors ist möglich.

Um die Bildung von Nebenprodukten bei der Reaktion zu vermeiden, ist es bevorzugt, Phosgen im Überschuss zuzuführen. Um nur den für die Reaktion notwendigen Anteil an Aminen zuzuführen, ist es möglich, das Amin mit einem Inertgas zu mischen. Durch den Anteil an Inertgas im Amin lässt sich die Menge des zugeführten Amins bei vorgegebener Geometrie der Zufuhröffnungen für das Amin und das Phosgen einstellen. Inertmedien, die zugegeben werden können, sind solche, die im Reaktionsraum gasförmig vorliegen und nicht mit den im Reaktionsverlauf auftretenden Verbindungen reagieren. Als Inertmedium können zum Beispiel Stickstoff, Edelgase wie Helium oder Argon, Aromaten wie Chlorbenzol, o-Dichlorbenzol, Trichlorbenzol, Toluol, Xylol, Chlornaphtalin, Decahydronaphtalin, Kohlenstoffdioxid oder Kohlenstoffmonoxid eingesetzt werden. Bevorzugt werden jedoch Stickstoff und/oder Chlorbenzol als Inertmedium verwendet.

Alternativ ist es jedoch auch möglich, zum Beispiel um einen zu großen Überschuss an Phosgen zu vermeiden, das Inertmedium dem Phosgen zuzumischen.

Im Allgemeinen wird das Inertmedium in einer Menge zugesetzt, dass das Verhältnis der Gasvolumina von Inertmedium zu Amin beziehungsweise zu Phosgen weniger als 0,0001 bis 30, bevorzugt weniger als 0,01 bis 15 und besonders bevorzugt weniger als 0,1 bis 5 beträgt.

Geeignete Beschichtungen, durch die ein Anhaften von auskondensierendem Amin verhindert werden kann, sind zum Beispiel solche, die SiOₓ enthalten, beispielsweise Silikosteel-Beschichtungen der Firma Restec Corporation.

Wenn das Auskondensieren von Amin an Oberflächen durch Beheizung der entsprechenden Oberflächen verhindert werden soll, so liegt die Temperatur, auf die die Oberfläche, die mit dem Amin in Kontakt kommen können, beheizt werden, vorzugsweise mindestens 5 K, mehr bevorzugt mindestens 10 K und insbesondere mindestens 15 K oberhalb der Kondensationsgrenze des Amin enthaltenden Gasgemischs. Die Kondensationsgrenze wird dabei durch den Druck in den Aminleitungen und den Inertgasanteil im Gasgemisch bestimmt.

Wenn beispielsweise der Anteil an Amin im Gasstrom erhöht wird oder die Vorrichtung zur Herstellung von Isocyanaten bei einem höheren Druck betrieben wird, ist es ebenfalls notwendig, auch die Temperaturen, auf die Oberflächen beheizt werden, die mit dem Amin in Kontakt kommen können, zu erhöhen.

Die Beheizung der Oberflächen kann dabei durch jedes beliebige, dem Fachmann bekannte Heizverfahren erfolgen. Bevorzugt zum Aufheizen der Oberflächen werden elektrische Heizelemente eingesetzt. Vorteil der Verwendung von elektrischen Heizelementen ist, dass sich diese auf einfache Weise auf eine vorgegebene Temperatur einstellen lassen.

Um die Bildung unerwünschter Nebenprodukte zu reduzieren beziehungsweise zu vermeiden und weiterhin auch eine Zersetzung des gebildeten Isocyanats zu unterbinden, wird das Reaktionsgas vorzugsweise unmittelbar nach der Reaktion in einem Quench abgekühlt. Hierzu wird ein im Allgemeinen flüssiges Quenchmedium zugegeben. Durch Verdampfung des Quenchmediums nimmt dieses Wärme auf und führt zu einer schnellen Abkühlung des Reaktionsgases. Durch die Zugabe des Quenchmediums entsteht ein Gemisch aus Reaktionsgas und Quenchmedium als Produktstrom.

Eine schnelle Abkühlung wird insbesondere dadurch erzielt, dass das Quenchmedium fein zerstäubt zugegeben wird. Hierdurch weist das Quenchmedium eine große Oberfläche auf und kann schnell die Wärme aufnehmen und damit das Reaktionsgas abkühlen.

Erfindungsgemäß wird das Quenchmedium flüssig mit einer Temperatur oberhalb der Kondensationstemperatur des Reaktionsgases zugegeben. Um eine vorzeitige Verdampfung des Quenchmediums zu vermeiden muss gegebenenfalls der Druck in der Zuleitung gegenüber dem Druck im Quenchraum erhöht sein. Die Entspannung auf den Druck des Quenchraums kann dann durch die Düsen selbst oder aber geeignete Regelorgane realisiert werden. Mit der Entspannung des Quenchmediums und Mischung mit den heißen Reaktionsgasen erfolgt eine Erwärmung und/oder eine teilweise oder vollständige Verdampfung des Quenchmediums. Die dabei aufgenommene Wärme führt zu einer Abkühlung der Reaktionsgase.

Insbesondere bei Verwendung eines Quenchmediums, das bei den Bedingungen im Quenchraum eine Siedetemperatur unterhalb der Kondensationstemperatur des Reaktionsgases aufweist, ist der Druck in den Zuleitungen gegenüber dem Druck im Quenchraum erhöht, um ein Verdampfen des Quenchmediums vor der Zugabe in den Quenchraum zu vermeiden.

Der Druck, mit dem das Quenchmedium zugegeben wird, liegt vorzugsweise im Bereich von 1 bis 20 bar, mehr bevorzugt im Bereich von 1 bis 10 bar und insbesondere im Bereich von 1 bis 8 bar.

Das zur Kühlung eingesetzte Quenchmedium enthält vorzugsweise ein Lösungsmittel, das ausgewählt ist aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophthalat, Tetrahydrofuran, Dimethylformamid, Xylol, Chlornaphthalin, Decahydronaphthalin und Toluol.

In einer Ausführungsform der Erfindung können sich an den Quench weitere Stufen zur Abkühlung des Reaktionsgases anschließen. In den einzelnen Stufen zur Abkühlung erfolgt dabei jeweils eine weitere Abkühlung des Reaktionsgases, bis zum Erreichen der gewünschten Endtemperatur, mit der das Reaktionsgas beispielsweise einer nachfolgenden Aufarbeitung zugeführt wird.

In einer Ausführungsform ist zumindest eine der sich an den Quench anschließenden Stufen zur Abkühlung des Reaktionsgases ein weiterer Quench.

So ist es zum Beispiel möglich, das den Quench und die sich gegebenenfalls daran anschließenden Stufen zur Abkühlung verlassende Reaktionsgas vorzugsweise bei Temperaturen von mehr als 130°C mit einem Lösungsmittel zu waschen. Als Lösungsmittel eignen sich zum Beispiel die gleichen Stoffe, die auch als Quenchmedium eingesetzt werden können.

Bei der Wäsche wird das Isocyanat selektiv in die Waschlösung überführt. Anschließend werden das verbleibende Gas und die erhaltene Waschlösung bevorzugt mittels Rektifikation in Isocyanat, Lösungsmittel, Phosgen und Chlorwasserstoff aufgetrennt.

Das Waschen des den Reaktor verlassenden Gasgemisches erfolgt vorzugsweise in einem Waschturm, wobei aus dem gasförmigen Gasgemisch das gebildete Isocyanat durch Kondensation in einem inerten Lösungsmittel abgetrennt wird, während überschüssiges Phosgen, Chlorwasserstoff und gegebenenfalls das Inertmedium die Aufarbeitungsvorrichtung gasförmig durchlaufen. Bevorzugt wird dabei die Temperatur des inerten Lösungsmittels oberhalb der Lösungstemperatur des zum Amin gehörigen Carbamoylchlorids im gewählten Waschmedium gehalten. Besonders bevorzugt wird dabei die Temperatur des inerten Lösungsmittels oberhalb der Schmelztemperatur des zum Amin gehörigen Carbamoylchlorids gehalten.

Die Wäsche kann in jeder beliebigen, dem Fachmann bekannten Vorrichtung durchgeführt werden. So eignen sich zum Beispiel Rührbehälter oder andere herkömmliche Apparaturen, beispielsweise Kolonnen oder Mixer-Settler-Apparaturen.

Das Waschen und die Aufarbeitung des den Reaktor verlassenden Reaktionsgases erfolgt dabei im Allgemeinen wie beispielsweise im WO-A 2007/028715 beschrieben.

Wie vorstehend bereits beschrieben, enthält eine Beschichtung vorzugsweise SiOₓ.

In einer weiteren bevorzugten Ausführungsform sind zudem Vorrichtungen zum Beheizen der Oberflächen, die mit gasförmigem Amin in Kontakt kommen können, umfasst. Als Vorrichtungen zum Beheizen der Oberflächen eignen sich insbesondere elektrische Heizelemente. Aber auch Ausführungsformen über Doppelmantelbeheizungen mit einem entsprechenden Heizmedium sind möglich. Oberflächen, die mit dem gasförmigem Amin in Kontakt kommen können, sind zum Beispiel Oberflächen des Verdampfers und des Reaktors sowie Wandungen von Rohren, die von dem gasförmigen Amin durchströmt werden. Durch die Verwendung von Vorrichtungen zum Beheizen der Oberflächen ist möglich, die Oberflächen auf einer Temperatur oberhalb der Kondensationstemperatur des Amin enthaltenden Gasgemischs zu halten und so ein Auskondensieren von Amin verhindern zu können. Hierdurch wird auch vermieden, dass sich Ablagerungen an den Oberflächen bilden.

## Patentansprüche

1. Verfahren zur Herstellung von Isocyanaten durch Umsetzung der korrespondierenden Amine mit Phosgen in der Gasphase, gegebenenfalls in Gegenwart eines Inertmediums, bei dem das Amin in einem Verdampfer zu einem Amin enthaltenden Gasstrom verdampft wird, das Phosgen in den Amin enthaltenden Gasstrom gemischt wird und das Amin und das Phosgen in einem Reaktor zum Isocyanat umgesetzt werden, **dadurch gekennzeichnet, dass** die Temperatur von mit dem gasförmigen Amin in Kontakt stehenden Oberflächen von Rohrleitungen, die den Verdampfer mit dem Reaktor verbinden, Zufuhrleitungen, Zufuhrdüsen oder Oberflächen des Verdampfers oberhalb der Taupunktsgrenze des Amin enthaltenden Gasstroms gehalten wird, wobei zum Halten der Temperatur der mit dem gasförmigen Amin in Kontakt stehenden Oberflächen oberhalb der Taupunktsgrenze des Amin enthaltenden Gasstroms Rohrleitungen und Apparate isoliert werden, um Wärmeverluste zu minimieren und zusätzlich mindestens eines der folgenden Merkmale erfüllt ist:
(a) Überhitzen des gasförmigen Aminstroms, um lokale Taupunktsunterschreitungen durch Wärmeverluste auszuschließen,
(b) Beheizen von Rohrleitungen und Apparaten, um Taupunktsunterschreitungen zu verhindern,
(c) Einspeisung eines temperierten Inertgasstroms, wobei die Temperatur des zugeführten Inertgases so eingestellt wird, dass ein Temperaturabstand der Mischtemperatur zur Kondensationsgrenze von mindestens 5 K besteht.

2. Verfahren gemäß Anspruch 1, **dadurch gekennzeichnet, dass** die Temperatur, auf die die Oberflächen, die mit dem Amin in Kontakt kommen können, beheizt werden, mindestens 5 K oberhalb der Kondensationstemperatur des das Amin enthaltenden Gasstroms liegt.

3. Verfahren gemäß Anspruch 1 oder 2, **dadurch gekennzeichnet, dass** zur Beheizung der Oberflächen elektrische Heizelemente oder Doppelmantelbeheizungen eingesetzt werden.

4. Verfahren gemäß einem der Ansprüche 1 bis 3, **dadurch gekennzeichnet, dass** durch die Einspeisung des temperierten Inertgasstroms ein Temperaturabstand von Mischtemperatur zur Kondensationsgrenze des das Amin enthaltenden Gasstroms von mindestens 5 K erzeugt wird.

5. Verfahren gemäß einem der Ansprüche 1 bis 4, **dadurch gekennzeichnet, dass** als Inertgas Stickstoff, Helium, Argon, Chlorbenzol, o-Dichlorbenzol, Toluol, Xylol, Chlornaphthalin, Decahydronaphthalin, Kohlenstoffdioxid, Kohlenstoffmonoxid oder Mischungen daraus eingesetzt werden.

6. Verfahren gemäß einem der Ansprüche 1 bis 5, **dadurch gekennzeichnet, dass** ein den Reaktor verlassendes, Isocyanat und Chlorwasserstoff enthaltendes Reaktionsgas in einem Quench durch Zugabe eines flüssigen Quenchmediums abgekühlt wird, wobei ein Gemisch aus Reaktionsgas und Quenchmedium als Produktstrom entsteht.

7. Verfahren gemäß Anspruch 6, **dadurch gekennzeichnet, dass** das Quenchmedium ein Lösungsmittel enthält, das ausgewählt ist aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophthalat, Tetrahydrofuran, Dimethylformamid, Xylol, Chlornaphthalin, Decahydronaphthalin und Toluol.

8. Verfahren gemäß Anspruch 5 oder 6, **dadurch gekennzeichnet, dass** sich an den Quench weitere Stufen zur Aufarbeitung des Produktstroms anschließen.

## Claims

1. A process for preparing isocyanates by reacting the corresponding amines with phosgene in the gas phase, optionally in the presence of an inert medium, in which the amine is evaporated in an evaporator to give an amine-comprising gas stream, the phosgene is mixed into the amine-comprising gas stream, and the amine and the phosgene are converted to the isocyanate in a reactor, wherein the temperature of surfaces of pipelines which connect the evaporator with the reactor, feed lines, feed nozzles or surfaces of the reactor that are in contact with the gaseous amine is kept above the dew point limit of the amine-comprising gas stream, keeping the temperature of the surfaces in contact with the gaseous amine above the dew point limit of the amine-comprising gas stream by insulating pipelines and apparatus in order to minimize heat losses and additionally fulfilling at least one of the following features:
(a) superheating the gaseous amine stream in order to rule out local dew point undershoots as a result of heat losses,
(b) heating of pipelines and apparatus in order to prevent dew point undershoots,
(c) feeding in an inert gas stream at controlled temperature, where the temperature of the supplied inert gas is adjusted such that there is a temperature margin of the mixing temperature from the condensation limit of at least 5 K.

2. The process according to claim 1, wherein the temperature to which the surfaces which can come into contact with the amine are heated is at least 5 K above the condensation temperature of the gas stream comprising the amine.

3. The process according to claim 1 or 2, wherein the surfaces are heated using electrical heating elements or jacket heaters.

4. The process according to any of claims 1 to 3, wherein feeding in the inert gas stream at a controlled temperature generates a temperature margin of mixing temperature from the condensation limit of the gas stream comprising the amine of at least 5 K.

5. The process according to any of claims 1 to 4, wherein the inert gas used is nitrogen, helium, argon, chlorobenzene, o-dichlorobenzene, toluene, xylene, chloronaphthalene, decahydronaphthalene, carbon dioxide, carbon monoxide or mixtures thereof.

6. The process according to any of claims 1 to 5, wherein a reaction gas which comprises isocyanate and hydrogen chloride and leaves the reactor is cooled in a quench by adding a liquid quench medium, which forms a mixture of reaction gas and quench medium as the product stream.

7. The process according to claim 6, wherein the quench medium comprises a solvent which is selected from the group consisting of monochlorobenzene, dichlorobenzene, trichlorobenzene, hexane, benzene, 1,3,5-trimethylbenzene, nitrobenzene, anisole, chlorotoluene, o-dichlorobenzene, diethyl isophthalate, tetrahydrofuran, dimethylformamide, xylene, chloronaphthalene, decahydronaphthalene and toluene.

8. The process according to claim 5 or 6, wherein the quench is followed by further stages for workup of the product stream.

## Revendications

1. Procédé de fabrication d'isocyanates par mise en réaction des amines correspondantes avec du phosgène en phase gazeuse, éventuellement en présence d'un milieu inerte, selon lequel l'amine est évaporée dans un évaporateur en un courant gazeux contenant l'amine, le phosgène est mélangé dans le courant gazeux contenant l'amine, et l'amine et le phosgène sont transformés dans un réacteur en l'isocyanate, **caractérisé en ce que** la température des surfaces en contact avec l'amine gazeuse des canalisations qui relient l'évaporateur avec le réacteur, des conduites d'alimentation, des buses d'alimentation ou des surfaces de l'évaporateur est maintenue au-dessus de la limite du point de condensation du courant gazeux contenant l'amine, les canalisations et les appareils étant isolés pour maintenir la température des surfaces en contact avec l'amine gazeuse au-dessus de la limite du point de condensation du courant gazeux contenant l'amine afin de minimiser les pertes de chaleur, et au moins une des caractéristiques suivantes étant en outre satisfaites :
(a) le surchauffage du courant d'amine gazeux afin d'exclure des diminutions locales en dessous du point de condensation par pertes de chaleur,
(b) le chauffage des canalisations et des appareils pour éviter des diminutions en dessous du point de condensation,
(c) l'introduction d'un courant de gaz inerte conditionné, la température du gaz inerte introduit étant ajustée de sorte qu'un écart de température entre la température de mélange et la limite de condensation d'au moins 5 K existe.

2. Procédé selon la revendication 1, **caractérisé en ce que** la température à laquelle les surfaces qui peuvent rentrer en contact avec l'amine sont chauffées est au moins 5 K au-dessus de la température de condensation du courant gazeux contenant l'amine.

3. Procédé selon la revendication 1 ou 2, **caractérisé en ce que** des éléments chauffants électriques ou des chauffages à double enveloppe sont utilisés pour le chauffage des surfaces.

4. Procédé selon l'une quelconque des revendications 1 à 3, **caractérisé en ce que** l'introduction du courant de gaz inerte conditionné génère un écart de température entre la température de mélange et la limite de condensation du courant gazeux contenant l'amine d'au moins 5 K.

5. Procédé selon l'une quelconque des revendications 1 à 4, **caractérisé en ce que** de l'azote, de l'hélium, de l'argon, du chlorobenzène, de l'o-dichlorobenzène, du toluène, du xylène, de la chloronaphtaline, de la décahydronaphtaline, du dioxyde de carbone, du monoxyde de carbone ou leurs mélanges sont utilisés en tant que gaz inerte.

6. Procédé selon l'une quelconque des revendications 1 à 5, **caractérisé en ce qu'**un gaz de réaction contenant de l'isocyanate et du chlorure d'hydrogène quittant le réacteur est refroidi dans une trempe par ajout d'un milieu de trempe liquide, un mélange de gaz de réaction et de milieu de trempe étant formé en tant que courant de produit.

7. Procédé selon la revendication 6, **caractérisé en ce que** le milieu de trempe contient un solvant qui est choisi dans le groupe constitué par le monochlorobenzène, le dichlorobenzène, le trichlorobenzène, l'hexane, le benzène, le 1,3,5-triméthylbenzène, le nitrobenzène, l'anisol, le chlorotoluène, l'o-dichlorobenzène, le diéthylisophtalate, le tétrahydrofurane, le diméthylformamide, le xylène, la chloronaphtaline, la décahydronaphtaline et le toluène.

8. Procédé selon la revendication 5 ou 6, **caractérisé en ce que** des étapes supplémentaires pour le traitement du courant de produit suivent la trempe.
